# DEMANDE DE BREVET EUROPEEN

(11) **EP 4 349 246 A1**
(43) Date de publication de la demande: **10.04.2024**
(21) Numéro de dépôt: 23201249.2
(22) Date de dépôt: 02.10.2023
(51) Int. Cl.: A61B 5/00, A61B 5/291, A61B 5/31

(54) **DISPOSITIF DE CAPTURE DE SIGNAUX EEG COMPRENANT UNE PLURALITÉ DE CAPTEURS RÉPARTIS SUR LA TÊTE**

(30) Priorité: 07.10.2022 FR 2210279
(71) Demandeur: Naixes, 13090 Aix-en-Provence (FR)
(72) Inventeur: CHARRAT, Bruno, 13090 AIX EN PROVENCE (FR)
(74) Mandataire: de Jong, Jean Jacques

(57) **Abrégé**

L'invention est relative à un dispositif de capture de signaux d'électroencéphalogramme EEG comprenant un capteur sans fil souple (10) fixé par adhésion au niveau d'un point de mesure d'EEG d'une tête d'un utilisateur, configuré pour être télé-alimenté et transmettre, par une première liaison radiofréquence (BT), des données EEG correspondant à des échantillons d'un signal d'EEG mesuré ; une station de base (12) disposée à proximité du capteur, configurée pour télé-alimenter le capteur ; et un terminal (16) configuré pour recevoir les données EEG transmises.

## Description

### Domaine technique

L'invention est relative à la mesure d'électroencéphalogrammes (EEG) pour déterminer des états cérébraux dans certaines activités humaines nécessitant un niveau élevé de concentration.

### Arrière-plan

Certains signaux EEG peuvent être utilisés pour quantifier des états cérébraux comme le niveau de relaxation, le stress, l'attention, la fatigue, la charge mentale, et même la préparation à une action musculaire.

De façon générale, selon le Système International 10-20, un EEG complet peut être réalisé à l'aide d'une vingtaine d'électrodes mises en contact électrique avec des points spécifiques du crâne, et sur lesquelles on mesure les tensions. On prévoit également une électrode servant de référence pour la mesure des tensions.

Pour déterminer certains états cérébraux, il suffit de relever les signaux EEG sur les deux points du front (nommés Fp1 et Fp2) et un point derrière chaque oreille (A1, A2).

Les électrodes sont généralement agencées dans des dispositifs portés sur la tête des utilisateurs, dont la configuration les rend peu propices à un usage en conditions réelles sur le terrain avec une personne en mouvement, hors d'un environnement d'expérimentation adapté.

### Résumé

On prévoit de façon générale un dispositif de capture de signaux d'électroencéphalogramme EEG comprenant un capteur sans fil souple fixé par adhésion au niveau d'un point de mesure d'EEG d'une tête d'un utilisateur, configuré pour être télé-alimenté et transmettre, par une première liaison radiofréquence, des données EEG correspondant à des échantillons d'un signal d'EEG mesuré ; une station de base disposée à proximité du capteur, configurée pour télé-alimenter le capteur ; et un terminal configuré pour recevoir les données EEG transmises.

La station de base peut être configurée pour recevoir les données EEG transmises par le capteur et retransmettre les données EEG par une deuxième liaison radiofréquence au terminal.

Selon une alternative, le terminal est configuré pour recevoir directement les données EEG transmises par le capteur.

Le capteur peut comprendre un substrat souple ; sur une première face du substrat, deux zones conductrices configurées pour être mises en contact électrique avec la peau et former deux électrodes d'un détecteur de signal d'EEG ; une piste conductrice sur une face du substrat, formant une antenne de télé-alimentation ; un circuit de commande agencé sur une deuxième face du substrat, relié par des pistes conductrices du substrat au détecteur d'EEG et à l'antenne de télé-alimentation, le circuit de commande étant configuré pour extraire son alimentation électrique du signal reçu par l'antenne de télé-alimentation, et numériser le signal issu du détecteur EEG et transmettre les données EEG correspondantes par la première liaison radiofréquence.

L'une des zones conductrices d'électrode peut être reliée à une masse du circuit de commande et l'autre zone conductrice d'électrode former un segment de la piste d'antenne, le circuit de commande étant configuré pour extraire l'alimentation et le signal d'EEG du signal de l'antenne.

La station de base peut être fixée à un dispositif porté sur la tête de l'utilisateur.

La station de base peut être configurée pour synchroniser l'échantillonnage des signaux d'EEG du capteur sans fil.

Le dispositif peut comprendre plusieurs capteurs sans fil, les capteurs sans fil coopérant pour qu'un capteur sans fil maître synchronise l'échantillonnage des signaux EEG de l'ensemble des capteurs sans fil.

La synchronisation peut utiliser des impulsions de synchronisation transmises par radiofréquence dans une bande de fréquences distincte de celle utilisée pour la transmission des données EEG et la télé-alimentation.

La station de base peut être configurée pour suspendre la télé-alimentation à des intervalles périodiques et le capteur sans fil être configuré pour acquérir au moins un échantillon du signal EEG dès que l'alimentation du capteur sans fil devient suffisante après une suspension.

La station de base peut être configurée pour interrompre la télé-alimentation à des intervalles périodiques de manière suffisamment brève pour ne pas interrompre l'alimentation du capteur sans fil, et le capteur sans fil être configuré pour synchroniser l'échantillonnage du signal EEG sur les intervalles d'interruption de la télé-alimentation.

Le dispositif peut comprendre plusieurs capteurs sans fil répartis sur les crânes de plusieurs utilisateurs, et plusieurs stations de base agencées dans des dispositifs respectifs portés sur les têtes des utilisateurs, chaque station de base étant configurée pour relayer au terminal déporté : les données EEG reçues des capteurs dans son champ de télé-alimentation, les données EEG reçues de capteurs hors de son champ de télé-alimentation, ou les données EEG reçues d'une autre station de base.

Les données EEG peuvent être transmises par le capteur ou la station de base par une liaison Wifi ou Bluetooth.

### Description sommaire des dessins

Des modes de réalisation seront exposés dans la description suivante, faite à titre non limitatif en relation avec les figures jointes parmi lesquelles :
La figure 1 illustre un mode de réalisation d'un dispositif de capture de signaux EEG comprenant une pluralité de capteurs sans fil répartis sur la tête ;
La figure 2A et la figure 2B représentent une vue de dessus et une vue en coupe d'un mode de réalisation de capteur EEG sans fil ni batterie ;
La figure 3 représente un schéma-bloc d'architecture électronique d'un dispositif de capture de signaux EEG utilisant un capteur sans fil selon les figures 2A et 2B ;
La figure 4 représente une vue de dessus d'un autre mode de réalisation de capteur EEG sans fil ;
La figure 5 représente un sous-ensemble analogique d'un circuit de commande du capteur de la figure 4 ;
La figure 6 illustre une mise en oeuvre de plusieurs capteurs sans fil répartis sur plusieurs personnes ;
La figure 7 représente un schéma-bloc d'architecture électronique d'un dispositif de capture de signaux EEG mettant en oeuvre un premier mode de synchronisation entre plusieurs capteurs EEG sans fil ayant un circuit de commande adapté ;
La figure 8 est un chronogramme illustrant un autre mode de synchronisation de plusieurs capteurs EEG sans fil ; et
La figure 9 est un chronogramme illustrant un autre mode de synchronisation de plusieurs capteurs EEG sans fil.

### Description détaillée

Dans la présente demande, on cherche à utiliser des états cérébraux déterminables au moyen de signaux EEG dans des applications visant l'amélioration de la performance mentale des personnes, comme l'amélioration par entraînement des temps de réaction d'un pilote de course, l'amélioration de la capacité de combat d'un pilote d'avion de chasse, la surveillance de l'état d'éveil d'un conducteur, l'amélioration de l'exécution d'une action de groupe (eSports, groupes d'intervention). Comme on l'a précédemment indiqué, les électrodes EEG sont classiquement incluses dans des dispositifs portés sur la tête des utilisateurs, qui ont une configuration qui les rend peu propices à un usage sur le terrain. En effet, ces dispositifs vont d'une coiffe dédiée rigide ou souple recouvrant l'ensemble du crâne et connectée par fils à une console déportée, à la bande élastique incluant un capteur dédié à un nombre limité de points rapprochés, alimenté par batterie, et communiquant avec une unité de traitement des données, comme un terminal ou smartphone.

Même le dispositif minimaliste sous forme de bande est inadapté à une utilisation sur le terrain. En effet, le module capteur avec son électronique de commande et son alimentation par batterie est encombrant du fait notamment de l'autonomie souhaitée, de sorte que la bande ne peut pas être portée sous un casque. De plus, le module est relativement lourd compte tenu de la taille de la batterie, de sorte que des chocs sur le terrain et des mouvements brusques de la tête peuvent déplacer le capteur.

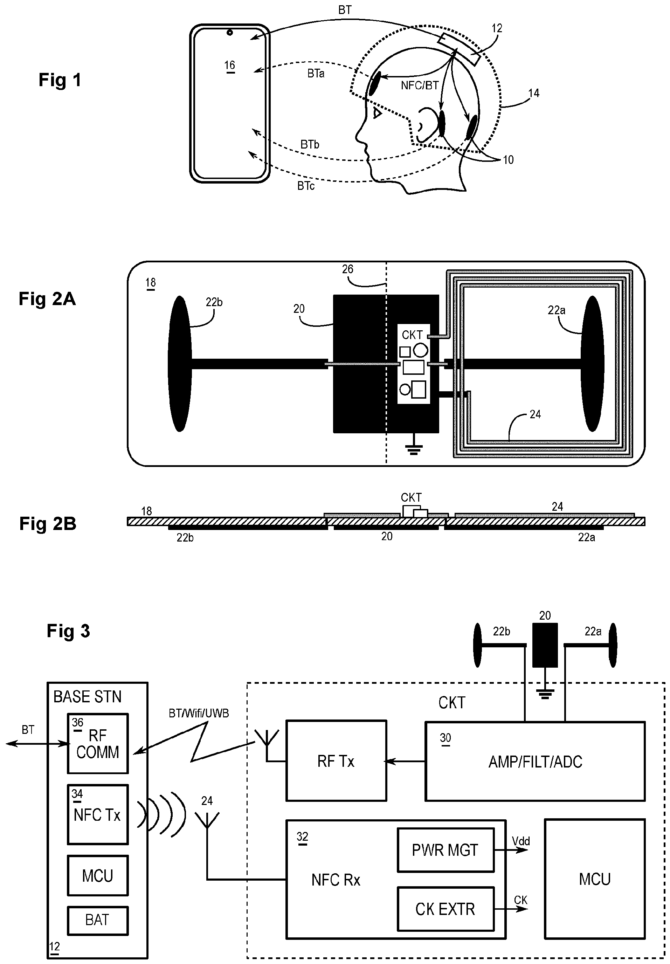

Dans la figure 1, pour pouvoir utiliser un ou plusieurs capteurs EEG sous un casque avec un risque réduit de déplacement intempestif, on prévoit des capteurs 10 sous forme de pastilles souples et minces à fixer par adhésion sur la peau, qui communiquent les données EEG par un lien radiofréquence (par exemple Bluetooth BT, Wifi, ou Ultra-Wide Band UWB), et qui sont télé-alimentés en permanence par une station de base proche 12 en utilisant une technique classique, comme la télé-alimentation en champ proche NFC ("Near-Field Communication") ou RFID (125Khz ou UHF). Ainsi, la technique de télé-alimentation, qui permet dans certains cas la transmission de données, sert ici seulement à l'alimentation des capteurs - les données EEG sont transmises par une autre voie, à savoir un lien radiofréquence de plus grande portée, de sorte que les contraintes de proximité liées à la transmission des données par NFC ou RFID ne s'appliquent pas.

La station de base 12 peut être intégrée dans un casque 14 et être alimentée par une batterie embarquée dans la station de base qui est moins soumise à des contraintes d'encombrement, ou déportée par fils dans un emplacement adapté, comme une poche de l'utilisateur, ou un boîtier fixé sur la paroi extérieure du casque.

Dans le cas d'un conducteur de véhicule, la station de base pourrait aussi être intégrée dans un volant, dans un appui-tête, ou dans le plafond au-dessus du conducteur.

Selon un mode de réalisation, la station de base 12 recueille les données transmises par les capteurs 10 et les communique à son tour à un terminal déporté 16, comme un smartphone, par une liaison sans fil Bluetooth BT ou Wifi, comme cela est représenté par des flèches BT en trait plein. Le terminal peut exécuter une application qui détermine et exploite les états cérébraux souhaités.

Dans certaines applications, le terminal pourrait être combiné avec la station de base, comme dans le cas d'un casque de réalité virtuelle (VR) dans lequel la station de base serait reliée comme un périphérique à l'unité de commande du casque VR, laquelle unité de commande est généralement reliée à un ordinateur par Wifi ou câble USB.

Selon un autre mode de réalisation, les capteurs 10 transmettent individuellement les données EEG directement au terminal 16 par leurs liens radiofréquence respectifs, comme cela est représenté par des flèches BTa à BTc en pointillés.

Dans ces configurations, la station de base 12 peut être amenée à alimenter des capteurs éloignés de 1 à 60 cm de la station de base, ce qui implique un dimensionnement adapté des antennes NFC. En fait, chaque capteur 10 envisagé ici est dédié à un seul point d'EEG, ou à une paire de points proches - il comprend ainsi pour chaque point deux électrodes, l'une placée en contact avec le point EEG correspondant, sur laquelle on mesure la tension (point chaud), et l'autre servant de référence (point froid). La position de l'électrode de référence peut être quelconque, mais elle est de préférence située à une distance minimale de 2 à 6 centimètres du point chaud. La taille résultante du capteur permet ainsi l'intégration d'une antenne de télé-alimentation de dimensions relativement grandes, de sorte qu'une télé-alimentation permanente peut être assurée par une station de base disposée jusqu'à quelques dizaines de centimètres des capteurs 10 collés sur le crâne de l'utilisateur. Lorsque la station de base est intégrée à un casque, la distance est inférieure à 10 cm.

Les figures 2A et 2B représentent une vue de dessus et une vue en coupe d'un mode de réalisation de capteur EEG sans fil télé-alimenté sous forme d'une pastille collable sur la peau. Le capteur représenté est adapté aux deux points du front ou de la nuque. Il comprend un substrat en matériau fin et souple 18, sensiblement rectangulaire. Le substrat peut être adapté à un procédé de dépôt de conducteurs en couche mince. Une première face du substrat, destinée à être collée sur la peau, porte des plages conductrices formant une électrode centrale de référence ou de masse 20 et deux électrodes symétriques 22a, 22b. La distance entre les électrodes 22a et 22b est de l'ordre de 5 cm.

La face opposée du substrat porte, sensiblement au centre, un circuit de commande CKT, réalisé par exemple à l'aide de circuits intégrés et des composants discrets fixés par de la colle conductrice à des pistes conductrices formées sur le substrat. Les électrodes 22a, 22b sont reliées au circuit CKT par des conducteurs s'étendant d'abord centralement sur la première face, puis traversant le substrat pour rejoindre le circuit de commande par la deuxième face.

Une antenne de télé-alimentation 24, par exemple ici de type NFC, comprend plusieurs spires d'un conducteur déposé sur l'une des faces du substrat, par exemple la deuxième face. L'extrémité externe de l'antenne est reliée au circuit de commande en restant sur la même face, tandis que l'extrémité interne traverse le substrat pour rejoindre l'électrode de masse 20 sur la première face.

Comme cela est représenté, l'antenne NFC est réalisée sur une moitié du substrat, et ses spires passent au plus près des bords du substrat, autour de l'électrode 22a. Le circuit de commande CKT est agencé sur la même moitié du substrat. Cette configuration asymétrique permet, lorsqu'un seul point EEG est à utiliser (comme derrière chaque oreille), de couper le capteur en deux selon une prédécoupe centrale 26, et d'utiliser seulement la moitié de droite, incluant le circuit et l'antenne 24. Ainsi on ne fabrique qu'un seul type de capteur pour une multitude de configurations de points de mesure EEG.

Lorsque le capteur est utilisé en entier, c'est-à-dire avec les deux électrodes 22a, 22b, le circuit de commande CKT gère individuellement deux signaux EEG, ce qui peut demander un surplus de puissance. Si la seule antenne 24 est insuffisante pour la télé-alimentation, on peut prévoir une antenne similaire sur la moitié gauche du capteur, connectée en parallèle sur l'antenne 24.

La figure 3 représente un schéma-bloc d'architecture électronique d'un dispositif de capture de signaux EEG utilisant un capteur sans fil selon les figures 2A et 2B. Le circuit de commande CKT comprend un sous-ensemble analogique 30 pour traiter les signaux reçus par les électrodes 22a, 22b. Le traitement peut inclure une amplification AMP, un filtrage FILT et une conversion en échantillons numériques ADC.

Les éléments numériques du capteur sont orchestrés par un microcontrôleur MCU. L'horloge et l'alimentation requises sont fournies par une unité d'extraction d'horloge CK EXTR et une unité de gestion d'alimentation PWR MGT faisant partie d'un récepteur NFC 32 opérant sur la porteuse de 13,56 MHz reçue sur l'antenne 24. Le récepteur NFC 32 peut être simplifié, puisqu'il n'a pas à traiter une réception de données.

Le microcontrôleur MCU traite les échantillons numériques pour produire des données EEG prêtes à émettre dans des trames de données selon un protocole standard de faible consommation et de courte portée, de préférence Bluetooth Low Energy (BLE). On peut aussi envisager une émission Wifi ou UWB. Un émetteur radiofréquence RF Tx correspondant reçoit les trames de données et les émet sur une antenne. L'émission peut être assurée par une antenne de petites dimensions par rapport à l'antenne NFC, sous forme également de conducteurs déposés en couche mince sur le substrat du capteur.

Le traitement des échantillons par le microcontrôleur MCU peut inclure diverses opérations, comme une compression de données par moyennage ou décimation pour réduire le débit et donc la consommation de l'émetteur.

La station de base 12 comprend un émetteur NFC simplifié 34, conçu pour n'émettre que la porteuse servant à la télé-alimentation.

Dans le cas où la station de base 12 est conçue pour recueillir les échantillons émis par les capteurs, celle-ci inclut une unité de communication radiofréquence 36 assurant la réception des échantillons et leur retransmission vers le terminal déporté 16. Les éléments de la station de base sont orchestrés par un microcontrôleur MCU et alimentés par une batterie BAT.

La portée d'émission requise dans ce cas étant particulièrement courte pour de la radiofréquence (au plus 20 cm), la puissance d'émission peut être abaissée significativement par rapport à des conditions nominales, diminuant d'autant la consommation en courant du capteur.

Dans le cas où les capteurs 10 transmettent les échantillons directement au terminal 16, la station de base 12 peut être dépourvue de l'unité de communication 36 et même du microcontrôleur MCU.

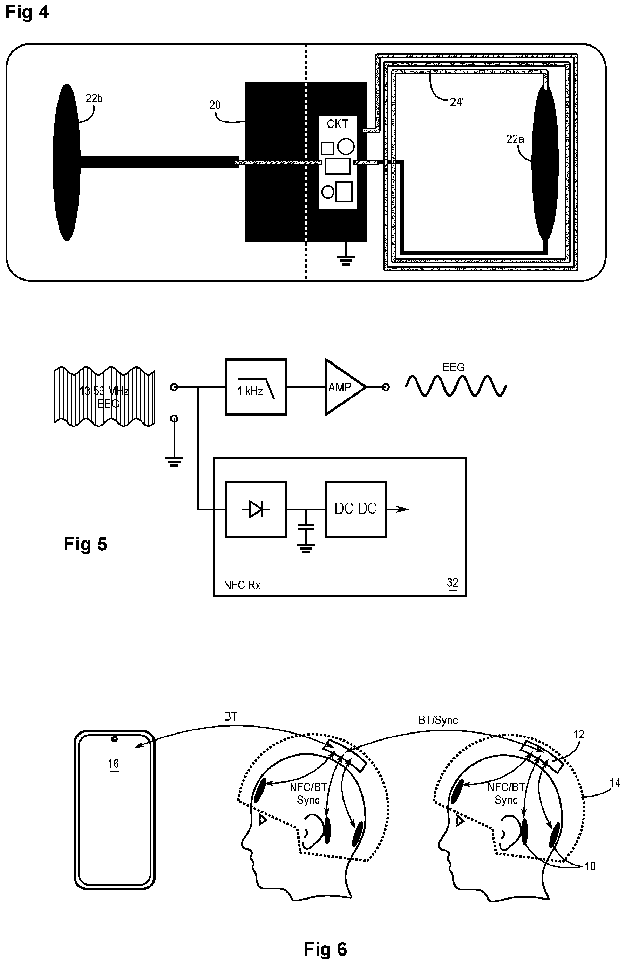

La figure 4 représente une vue de dessus d'un autre mode de réalisation de capteur EEG sans fil. Par rapport au capteur de la figure 2A, l'électrode de droite 22a' forme un segment de la piste de l'antenne NFC 24'. De préférence, l'électrode 22a' se trouve du côté du point chaud de l'antenne, de sorte que la plus grande longueur de la piste d'antenne est reliée entre l'électrode et la masse, comme cela est représenté. Ainsi, à la fréquence de la porteuse NFC, la longueur de piste entre l'électrode et la masse forme une impédance élevée, limitant l'atténuation du signal EEG reçu sur l'électrode 22a'.

La figure 5 représente un sous-ensemble analogique du circuit de commande CKT, apte à traiter le signal de l'antenne 24' du capteur de la figure 4. Comme cela est représenté à gauche, le signal EEG module l'amplitude de la porteuse NFC de 13,56 MHz. Ce signal modulé alimente deux voies, une première servant à extraire de façon classique le courant d'alimentation dans le récepteur NFC 32, à l'aide d'un redresseur, un condensateur de filtrage, et un convertisseur continu-continu. Une deuxième voie effectue un filtrage passe-bas adapté aux signaux EEG, avec une fréquence de coupure de 1 kHz, par exemple, et une amplification.

La figure 6 illustre une mise en oeuvre de plusieurs capteurs sans fil répartis sur plusieurs personnes. Plusieurs utilisateurs sont équipés, chacun, d'un jeu de capteurs EEG 10 et d'une station de base correspondante 12, de préférence intégrée dans un casque 14, comme à la figure 1. Dans le cas où les stations de base 12 sont conçues pour relayer les données EEG des capteurs, les capacités de communication par radiofréquence des stations de base sont redondantes. En effet, chaque station de base 12 peut être configurée pour transmettre individuellement ses échantillons à un terminal déporté commun 16, ou bien, comme cela est représenté, les stations de base peuvent être chaînées pour que chacune transmette ses échantillons à une autre station de base. Alors, une dernière station de base de la chaîne transmet tous les échantillons recueillis au terminal 16. Une station de base d'une première personne peut même recevoir les échantillons émis par les capteurs d'une autre personne à proximité. Les stations de base peuvent donc être conçues pour se reconfigurer dynamiquement en fonction des liaisons les plus performantes pour optimiser la consommation et diminuer les erreurs.

Dans le cas où les capteurs 10 sont configurés pour transmettre individuellement leurs données EEG au terminal 16, les stations de base 12 ne communiquent pas entre elles ou avec le terminal, ce qui simplifie la gestion.

Compte tenu de la configuration des capteurs 10, chaque capteur est indépendant et produit ses échantillons de manière asynchrone. Ainsi, si les multiples capteurs d'un dispositif démarrent à des instants différents, ou subissent des dérives d'horloge du fait que les horloges sont fournies par des stations de base différentes, la correspondance temporelle entre d'échantillons reçus de capteurs différents peut être perdue, ce qui peut induire des erreurs d'interprétation. On prévoit ci-après plusieurs possibilités de synchronisation des acquisitions des signaux EEG des capteurs.

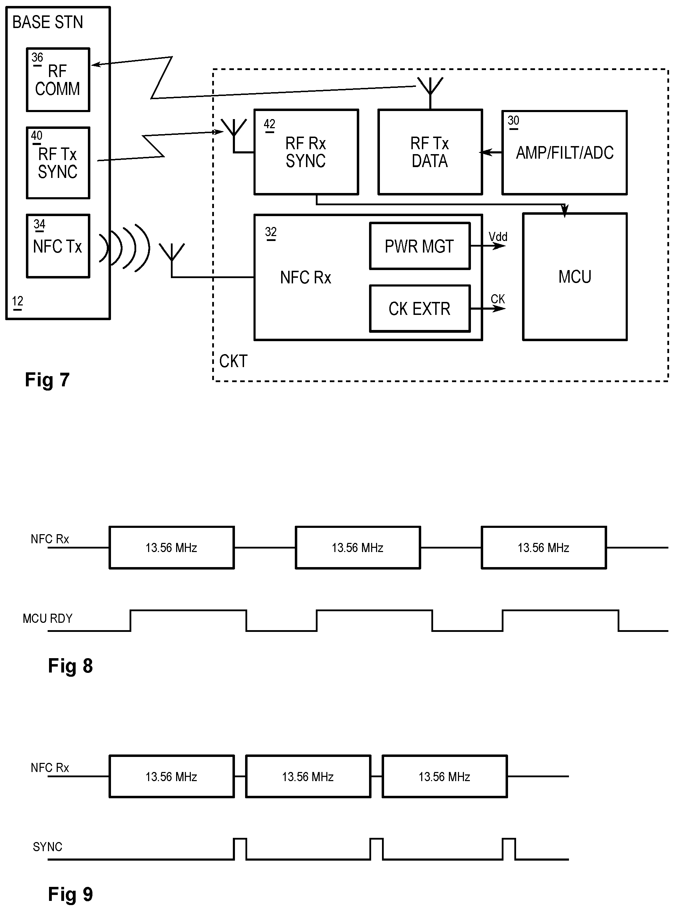

La figure 7 représente un schéma-bloc d'architecture électronique d'un dispositif de capture de signaux EEG mettant en oeuvre un premier mode de synchronisation des capteurs. Chaque station de base 12 comprend un émetteur d'impulsions de synchronisation 40, utilisant une bande radiofréquence bas débit et basse consommation. On peut utiliser, par exemple, la bande de fréquences 433-434 MHz servant aux télécommandes radio très basse consommation, compte tenu du faible nombre d'informations à transmettre (comme une impulsion ou un numéro de séquence).

Chaque capteur intègre alors un récepteur de synchronisation 42 correspondant qui extrait les impulsions de synchronisation. Les impulsions de synchronisation peuvent interrompre le microcontrôleur MCU, programmé alors pour provoquer l'acquisition d'un ou plusieurs échantillons à chaque impulsion de synchronisation.

Dans la situation impliquant plusieurs personnes (figure 6), chacune des stations de base aurait la capacité de synchroniser les capteurs. Dans ce cas, de préférence, une seule station de base maître est utilisée pour la synchronisation. La portée des signaux de synchronisation reste suffisamment longue pour couvrir plusieurs personnes agissant en groupe. Le cas échéant, la station de base maître est choisie en fonction de sa position centrale dans le groupe.

Selon une variante, un capteur 10 maître émet les impulsions de synchronisation pour synchroniser tous les autres capteurs.

La figure 8 est un chronogramme illustrant un autre mode de synchronisation de plusieurs capteurs. La station de base 12 est configurée pour suspendre périodiquement la porteuse (signal NFC Rx) pendant un intervalle fixe. Chaque capteur 10 voit ainsi son alimentation cesser et reprendre périodiquement. Le capteur est alors configuré pour que la première chose faite au rétablissement de l'alimentation soit d'acquérir un ou plusieurs échantillons. La durée de l'intervalle d'alimentation est suffisamment longue pour que le capteur ait le temps de transmettre ensuite les échantillons à la station de base. Le rapport cyclique de la séquence d'alimentation peut être choisi pour optimiser la puissance consommée de la station de base.

Le signal MCU RDY illustre les phases où l'alimentation est suffisante pour le microcontrôleur. Ces phases sont décalées par rapport aux rétablissements de la porteuse du fait que la tension d'alimentation doit à chaque fois atteindre un niveau suffisant pour alimenter le microcontrôleur, et que le microcontrôleur doit ensuite exécuter une phase de démarrage avant de procéder à l'acquisition. On peut en principe compter sur le fait que ce décalage est constant ou varie peu.

Ce mode de synchronisation s'applique seulement entre une station de base et les capteurs qui sont à portée de télé-alimentation. Pour synchroniser les capteurs de plusieurs personnes, on peut envisager que les stations de base 12 se synchronisent entre elles en utilisant les liaisons de communication RF servant à relayer les échantillons, ou des liaisons RF basse consommation dédiées (ex. 433 MHz).

La figure 9 est un chronogramme illustrant un autre mode de synchronisation de plusieurs capteurs. La station de base 12 est configurée pour interrompre périodiquement la porteuse (signal NFC Rx) pendant un intervalle suffisamment bref pour que l'alimentation des circuits du capteur ne soit pas interrompue, grâce notamment au relai temporaire assuré par les condensateurs de filtrage de l'alimentation. Le circuit de réception NFC de chaque capteur 10 peut être configuré pour extraire une deuxième horloge de ces intervalles d'interruption, produisant ainsi un signal de synchronisation SYNC. Le signal SYNC peut être exploité comme les impulsions de synchronisation transmises par RF dans le mode de réalisation de la figure 7.

Comme pour la figure 8, ce mode de synchronisation s'applique seulement entre une station de base et les capteurs qui sont à portée de télé-alimentation. Pour synchroniser les capteurs de plusieurs personnes, on peut envisager que les stations de base 12 se synchronisent entre elles en utilisant les liaisons de communication RF servant à relayer les échantillons, ou des liaisons RF basse consommation dédiées (ex. 433 MHz).

## Revendications

1. Dispositif de capture de signaux d'électroencéphalogramme EEG comprenant :
un capteur sans fil souple (10) fixé par adhésion au niveau d'un point de mesure d'EEG d'une tête d'un utilisateur, configuré pour être télé-alimenté et transmettre, par une première liaison radiofréquence (BT), des données EEG correspondant à des échantillons d'un signal d'EEG mesuré ;
une station de base (12) disposée à proximité du capteur, configurée pour télé-alimenter le capteur ; et
un terminal (16) configuré pour recevoir les données EEG transmises.

2. Dispositif selon la revendication 1, dans lequel la station de base (12) est configurée pour recevoir les données EEG transmises par le capteur (10) et retransmettre les données EEG par une deuxième liaison radiofréquence (BT) au terminal (16).

3. Dispositif selon la revendication 1, dans lequel le terminal (16) est configuré pour recevoir directement les données EEG transmises par le capteur (10).

4. Dispositif selon la revendication 1, dans lequel capteur (10) comprend :
un substrat souple (18) ;
sur une première face du substrat, deux zones conductrices (20, 22a) configurées pour être mises en contact électrique avec la peau et former deux électrodes d'un détecteur de signal d'EEG ;
une piste conductrice (24) sur une face du substrat, formant une antenne de télé-alimentation ;
un circuit de commande (CKT) agencé sur une deuxième face du substrat, relié par des pistes conductrices du substrat au détecteur d'EEG et à l'antenne de télé-alimentation, le circuit de commande étant configuré pour :
extraire son alimentation électrique (NFC Rx) du signal reçu par l'antenne de télé-alimentation ; et
numériser le signal issu du détecteur EEG et transmettre les données EEG correspondantes par la première liaison radiofréquence (RF Tx).

5. Dispositif selon la revendication 4, dans lequel l'une des zones conductrices d'électrode (20) est reliée à une masse du circuit de commande (CKT) et l'autre zone conductrice d'électrode (22a) forme un segment de la piste d'antenne, le circuit de commande étant configuré pour extraire l'alimentation et le signal d'EEG du signal de l'antenne.

6. Dispositif selon la revendication 1, dans lequel la station de base est fixée à un dispositif (14) porté sur la tête de l'utilisateur.

7. Dispositif selon la revendication 1, dans lequel la station de base est configurée pour synchroniser l'échantillonnage des signaux d'EEG du capteur sans fil.

8. Dispositif selon la revendication 1 comprenant plusieurs capteurs sans fil (10), les capteurs sans fil coopérant pour qu'un capteur sans fil maître synchronise l'échantillonnage des signaux EEG de l'ensemble des capteurs sans fil.

9. Dispositif selon la revendication 7 ou 8, dans lequel la synchronisation utilise des impulsions de synchronisation transmises par radiofréquence dans une bande de fréquences distincte de celle utilisée pour la transmission des données EEG et la télé-alimentation.

10. Dispositif selon la revendication 7, dans lequel la station de base est configurée pour suspendre la télé-alimentation à des intervalles périodiques et le capteur sans fil est configuré, pour acquérir au moins un échantillon du signal EEG dès que l'alimentation du capteur sans fil devient suffisante après une suspension.

11. Dispositif selon la revendication 7, dans lequel la station de base est configurée pour interrompre la télé-alimentation à des intervalles périodiques de manière suffisamment brève pour ne pas interrompre l'alimentation du capteur sans fil, et le capteur sans fil est configuré pour synchroniser l'échantillonnage du signal EEG sur les intervalles d'interruption de la télé-alimentation.

12. Dispositif selon la revendication 2, comprenant plusieurs capteurs sans fil (10) répartis sur les crânes de plusieurs utilisateurs, et plusieurs stations de base (12) agencées dans des dispositifs respectifs (14) portés sur les têtes des utilisateurs, chaque station de base étant configurée pour relayer au terminal déporté (16) :
les données EEG reçues des capteurs dans son champ de télé-alimentation,
les données EEG reçues de capteurs hors de son champ de télé-alimentation, ou
les données EEG reçues d'une autre station de base.

13. Dispositif selon la revendication 1, dans lequel les données EEG sont transmises par le capteur ou la station de base par une liaison Wifi ou Bluetooth.
